## Europäisches Patentamt
## European Patent Office
## Office européen des brevets

(11) Publication number: **0 125 594**
**B1**

(12) **EUROPEAN PATENT SPECIFICATION**

(45) Date of publication of patent specification: **03.10.90**

(21) Application number: **84105109.7**

(22) Date of filing: **05.05.84**

(51) Int. Cl.⁵: **A 61 K 31/57,** A 61 K 9/06, A 61 K 47/00

(54) Betamethasone dipropionate cream.

(30) Priority: 13.05.83 US 494214
10.11.83 US 550434

(43) Date of publication of application:
21.11.84 Bulletin 84/47

(45) Publication of the grant of the patent:
03.10.90 Bulletin 90/40

(84) Designated Contracting States:
AT BE CH DE FR GB IT LI NL SE

(56) References cited:
EP-A-0 068 553
FR-A-2 126 383
US-A-4 008 321
US-A-4 070 462

H. Janistyn, H. der Kosmetica und Riechstoffe
(1978) p.936

The file contains technical information
submitted after the application was filed and
not included in this specification

(73) Proprietor: SCHERING CORPORATION
2000 Galloping Hill Road
Kenilworth New Jersey 07033 (US)

(72) Inventor: Sandweiss, Varda Ecker
109-33 71 Road
Forest Hills New York 11375 (US)
Inventor: Stupak, Elliot
1 Essex Place
West Caldwell New Jersey 07006 (US)
Inventor: Shapiro, Paul Hyman
17 Rosemere Avenue
West Caldwell New Jersey 07006 (US)

(74) Representative: von Kreisler, Alek, Dipl.-Chem.
et al
Deichmannhaus am Hauptbahnhof
D-5000 Köln 1 (DE)

Courier Press, Leamington Spa, England.

## Description

This invention relates to an elegant cream-like formulation of betamethasone dipropionate as defined by the claims.

A betamethasone dipropionate cream has been sold by Schering Corporation. That product consists of betamethasone dipropionate in a cream base consisting of purified water, mineral oil, white petrolatum, polyethylene glycol 1000 monocetyl ether, cetostearyl alcohol, monobasic sodium phosphate and phosphoric acid, with 4-chloro-m-cresol and propylene glycol as preservatives.

The formulation of the present invention is remarkably simple compared to this known cream. Since it contains a glycol solvent as a minor component, other preservatives may be omitted as may surfactants. It is thus likely to cause allergic reactions due to these additives. Nevertheless, the formulation of the present invention is highly efficacious and is stable. In fact, it is surprisingly more effective than the said known cream. Also, even though standard preservatives are not present, the present formulation has been found to be self preserving when tested in standardized assays for the determination of growth of *Escherichia coli, Candida albicans, Staphylococcus aureus, Aspergillis niger,* and *Pseudomonoas aeruginosa.* In standard dermal irritation studies on rabbits, the formulation of the present invention has been found to be less irritating than the said known cream.

The formulation of this invention is of value in the topical treatment of dermatological disorders that are responsive to corticosteroids. Included within this category are disorders such as psoriasis, contact dermatitis, atopic dermatitis and eczema. Treatment with the formulation of this invention is usually accomplished by applying the formulation to completely cover the affected area. The usual frequency of application is twice daily, although adequate maintenance therapy for some patients may be achieved with less frequent application.

The formulation of the present invention consists of 0.02 to 0.10 percent betamethasone dipropionate, zero to 0.6 percent titanium dioxide, 1.0 to 2.5 percent Carbomer 940 which is a polymer of acrylic acid crosslinked with a polyfunctional agent as defined below sufficient base to maintain the pH to between pH 4.0 and pH 5.5, and 60 to 80 percent of propylene glycol and water to make 100 percent (all percentages are by weight).

In a preferred aspect of the present invention, 0.06 to 0.08 percent betamethasone dipropionate is employed and 65 to 75 percent of propylene glycol. It is also useful if the composition contains 0.4 to 0.6 percent titanium dioxide.

Suitable bases are organic and preferably inorganic bases. The preferred base is sodium hydroxide.

Carbomer 940 is an acrylic acid polymer having an approximate molecular weight of 4,000,000. It is available from B. F. Goodrich Chemical Company as Carbopol 940 resin.

The formulation of this invention results in an elegant cream-like gel when it contains from 0.4 to 0.6 percent titanium dioxide. If no titanium dioxide is used, a clear consistency is attained. The use of the former formulation is particularly advantageous as such formulation has the desired appearance of a cream and is more easily applied than prior art gels while it is surprisingly more effective than the discussed cream known in the art.

The formulation of the present invention is manufactured in a conventional manner by thoroughly mixing the ingredients at ambient or elevated temperatures.

Preferably, the betamethasone dipropionate, dissolved in a portion of propylene glycol, is added to the cream-base. The ingredients are thoroughly mixed so that the product is homogeneous. If desired, additional mechanical agitation can be used as an intermediate or final step in the manufacturing process to impart more homogeneity or improve texture. Processing equipment suitable for these steps is known and includes heat exchangers, propeller mixers, colloid mills, homogenizers or roller mills.

The following formulation examples illustrate the compositions of the present invention. It will be apparent to those skilled in the art that many modifications thereof may be practiced without departing from the purpose and intent of this disclosure.

## Examples

| Betamethasone Dipropionate Cream Ingredients | Formulation (mg/g) | | |
|---|---|---|---|
| | I | II | III |
| Betamethasone 17,21-dipropionate USP | 0.7 | 0.7 | 1.0 |
| Titanium Dioxide USP | 5.0 | 5.0 | 5.0 |
| Carbomer 940* | 20.0 | 15.0 | 15.0 |
| Sodium Hydroxide USP | 0.4 | 0.3 | 0.3 |
| Propylene Glycol USP | 700.0 | 700.0 | 800.0 |
| Purified Water USP | 273.9 | 279.0 | 178.7 |

The ingredients are thoroughly mixed according to known methods.

*Carbomer 940 is a polymer of acrylic acid crosslinked with a polyfunctional agent; CAS Number: 9003—01—4 (TSCA); 9007—17—4; (CTFA Cosmetic Ingredient Dictionary Third Edition; Published by The Cosmetic, Toiletry and Fragrance Asociation, Inc., 1110 Vermont Avenue, N.W., Washington, D.C. 20005).

**Claims for the Contracting States: BE CH DE FR GB IT LI NL SE**

1. A topical pharmaceutical formulation for the treatment of inflammation consisting of 0.02 to 0.10 percent betamethasone dipropionate; 0.4 to 0.6 percent titanium dioxide; 1.0 to 2.5 percent of a polymer of acrylic acid crosslinked with a polyfunctional agent; sufficient base to adjust the pH to between pH 4.0 to pH 5.5; and 60 to 80 percent of propylene glycol and water to make 100 percent.

2. A pharmaceutical formulation according to claim 1 consisting of 0.06 to 0.08 percent betamethasone dipropionate; 0.4 to 0.6 percent titanium dioxide; 1.0 to 2.5 percent of polymer of acrylic acid crosslinked with a polyfunctional agent; sufficient base to adjust the pH to between pH 4.0 to pH 5.5; and 65 to 75 percent of propylene glycol and water to make 100 percent.

3. A pharmaceutical formulation according to claim 1 or 2 wherein a polymer of acrylic acid crosslinked with a polyfunctional agent is present in an amount of 2%.

4. A pharmaceutical formulation according to any one of claims 1 to 3, wherein said base is sodium hydroxide.

5. Process for the preparation of a formulation according to any one of claims 1 to 4 which consists in mixing the ingredients specified in any one of claims 1 to 4.

6. Use of a pharmaceutical formulation as claimed in claims 1 to 4 and 5 for the manufacture of an anti-inflammatory medicament.

**Claims for the Contracting State: AT**

1. Process for the preparation of a topical pharmaceutical formulation for the treatment of inflammation which consists in mixing 0.02 to 0.10 percent betamethasone dipropionate; 0.4 to 0.6 percent titanium dioxide; 1.0 to 2.5 percent of polymer crosslinked with a polyfunctional agent, sufficient base to adjust the pH to between pH 4.0 to pH 5.5; and 60 to 80 percent of propylene glycol and water to make 100 percent.

2. Process according to claim 1, consisting in mixing 0.06 to 0.08 percent betamethasone dipropionate; 0.4 to 0.6 percent titanium dioxide; 1.0 to 2.5 percent of a polymer crosslinked with a polyfunctional agent; sufficient base to adjust the pH to between pH 4.0 to pH 5.5; and 65 to 75 percent of propylene glycol and water to make 100 percent.

3. Process according to claim 1 or 2 wherein an acrylic polymer crosslinked with a polyfunctional agent is admixed in an amount of 2%.

4. Process according to any one of claims 1 to 3, wherein said base is sodium hydroxide.

**Patentansprüche für die Vertragsstaaten: BE CH DE FR GB IT LI LU NL SE**

1. Topische pharmazeutische Formulierung zur Behandlung von Entzündungen, bestehend aus 0,02 bis 0,10% Betamethason-dipropionat, 0,4 bis 0,6% Titandioxid, 1,0 bis 2,5% eines Polymers aus Acrylsäure, das mit einem polyfunktionellen Mittel vernetzt ist, ausreichende Mengen einer Base, um den pH-Wert auf einen Wert zwischen 4,0 und 5,5 einzustellen, und 60 bis 80% Propylenglycol, und Wasser auf 100%.

2. Pharmazeutische Formulierung nach Anspruch 1, bestehend aus 0,06 bis 0,08% Betamethason-dipropionat, 0,4 bis 0,6% Titandioxid, 1,0 bis 2,5% eines Polymers aus Acrylsäure, das mit einem polyfunktionellen Mittel vernetzt ist, ausreichende Mengen einer Base, um den pH-Wert auf einen Wert zwischen 4,0 und 5,5 einzustellen, und 65 bis 75% Propylenglycol, und Wasser auf 100%.

3. Pharmazeutische Formulierung nach den Ansprüchen 1 oder 2, worin das Polymer aus Acrylsäure, das mit einem polyfunktionellen Mittel vernetzt ist, in einer Menge von 2% vorhanden ist.

4. Pharmazeutische Formulierung nach irgendeinem der Ansprüche 1 bis 3, worin die Base Natriumhydroxid ist.

5. Verfahren zur Herstellung einer Formulierung nach irgendeinem der Ansprüche 1 bis 4, bestehend aus dem Vermischen der in irgendeinem der Ansprüche 1 bis 4 spezifizierten Bestandteile.

6. Verwendung einer Formulierung nach irgendeinem der Ansprüche 1 bis 4 oder hergestellt nach Anspruch 5 zur Herstellung eines entzündungshemmenden Medikaments.

**Patentansprüche für den Vertragsstaat: AT**

1. Verfahren zur Herstellung einer topischen pharmazeutischen Formulierung zur Behandlung von Entzündungen, umfassend das Vermischen von 0,02 bis 0,10% Betamethason-dipropionat, 0,4 bis 0,6% Titandioxid, 1,0 bis 2,5% eines Polymers aus Acrylsäure, das mit einem polyfunktionellen Mittel vernetzt ist, ausreichende Mengen einer Base, um den pH-Wert auf einen Wert zwischen 4,0 und 5,5 einzustellen, und 60 bis 80% Propylenglycol, und Wasser auf 100%.

2. Verfahren nach Anspruch 1, umfassend das Vermischen von 0,06 bis 0,08% Betamethason-dipropionat, 0,4 bis 0,6% Titandioxid, 1,0 bis 2,5% eines Polymers aus Acrylsäure, das mit einem polyfunktionellen Mittel vernetzt ist, ausreichende Mengen einer Base, um den pH-Wert auf einen Wert zwischen 4,0 und 5,5 einzustellen, und 65 bis 75% Propylenglycol, und Wasser auf 100%.

3. Verfahren nach den Ansprüchen 1 oder 2, worin ein Polymer aus Acrylsäure, das mit einem polyfunktionellen Mittel vernetzt ist, in einer Menge von 2% zugemischt wird.

4. Verfahren nach irgendeinem der Ansprüche 1 bis 3, worin die Base Natriumhydroxid ist.

**Revendications pour l'Etats contractants: BE CH DE FR GB IT LI LU NL SE**

1. Formule pharmaceutique topique pour le traitement d'une inflammation, consistant en 0,02 à 0,10 pour cent de dipropionate de bétaméthasone, 0,4 à 0,6 pour cent de bioxyde de titane; 1,0 à 2,5 pour cent d'un polymère d'acide acrylique réticulé par un agent polyfonctionnel; suffisamment de base pour ajuster le pH entre 4,0 et 5,5; et 60 à 80 pour cent de propylène glycol et de l'eau pour obtenir 100 pour cent.

2. Formule pharmaceutique selon la revendication 1, consistant en 0,06 à 0,08 pour cent de dipropionate de bétaméthasone; 0,4 à 0,6 pour cent de bioxyde de titane; 1,0 à 2,5 pour cent d'un polymère d'acide acétique réticulé avec un agent polyfonctionnel; suffisamment de base pour ajuster le pH entre 4,0 et 5,5; et 65 à 75 pour cent de propylène glycol et de l'eau pour obtenir 100 pour cent.

3. Formule pharmaceutique selon la revendication 1 ou 2, où le polymère d'acide acrylique réticulé avec un agent polyfonctionnel est présent en une quantité de 2%.

4. Formule pharmaceutique selon l'une quelconque des revendications 1 à 3, où ladite base est de l'hydroxyde de sodium.

5. Procédé pour la préparation d'une formule selon l'une quelconque des revendications 1 à 4, qui consiste à mélanger les ingrédients spécifiés selon l'une quelconque des revendications 1 à 4.

6. Utilisation d'une formule pharmaceutique selon les revendications 1 à 4 et 6 pour la fabrication d'un médicament anti-inflammatoire.

**Revendications pour l'Etat contractant: AT**

1. Procédé de préparation d'une formule pharmaceutique topique pour le traitement d'une inflammation, qui comprend le mélange de 0,02 à 0,10 pour cent de dipropionate de bétaméthasone; de 0,4 à 0,6 pour cent de bioxyde de titane; de 1,0 à 2,5 pour cent d'un polymère d'acide acrylique réticulé par un agent polyfonctionnel; suffisamment d'une base pour ajuster le pH entre 4,0 et 5,5; et 60 à 80 pour cent de propylène glycol et de l'eau pour obtenir 100 pour cent.

2. Procédé selon la revendication 1, comprenant le mélange de 0,06 à 0,08 pour cent de dipropionate de bétaméthasone; de 0,4 à 0,6 pour cent de bioxyde de titane; de 1,0 à 2,5 pour cent d'un polymère d'acide acrylique réticulé par un agent polyfonctionnel; suffisamment de base pour ajuster le pH entre 4,0 et 5,5; et 65 à 75 pour cent de propylène glycol et de l'eau pour obtenir 100 pour cent.

3. Procédé selon la revendication 1 ou 2, ou un polymère d'acide acrylique réticulé par un agent polyfonctionnel est mélangé en une quantité de 2%.

4. Procédé selon l'une quelconque des revendications 1 à 3, où ladite base est de l'hydroxyde de sodium.